# EUROPEAN PATENT APPLICATION

(11) **EP 4 628 010 A1**
(43) Date of publication of application: **08.10.2025**
(21) Application number: 23897233.5
(22) Date of filing: 22.09.2023
(51) Int. Cl.: A61B 5/11, A61B 5/22, A61C 19/04, A61C 19/045

(54) **CHEWING ABILITY MEASUREMENT METHOD, CHEWING ABILITY CONFIRMATION SYSTEM, AND DEDICATED MOUNT**

(30) Priority: 28.11.2022 JP 2022189656
(71) Applicant: Lotte Co., Ltd., Tokyo 160-0023 (JP)
(72) Inventor: SAKANOSHITA, Norimasa, Tokyo 160-0023 (JP); HIROTA, Yusuke, Saitama-shi, Saitama 336-8601 (JP); SEKI, Tetsuya, Saitama-shi, Saitama 336-8601 (JP); OKABAYASHI, Kazuto, Saitama-shi, Saitama 336-8601 (JP); EMURA, Koji, Saitama-shi, Saitama 336-8601 (JP); KINOSHITA, Shigenori, Tokyo 135-0061 (JP)
(74) Representative: Witte, Weller & Partner Patentanwälte mbB
(86) International application number: PCT/JP2023/034581
(87) International publication number: WO 2024/116574

(57) **Abstract**

A method capable of objectively, accurately and readily measuring the color of a post-chew chewing check gum is desired. The post-chew chewing check gum is placed on a dedicated mount 10 provided with chromatic reference colors 12, and the dedicated mount 10 is photographed by a camera. A photographed image of the chromatic reference colors 12 is compared with the chromatic reference colors 12 provided on the dedicated mount 10, and a color correction amount of the photographed chromatic reference color image is calculated. Then, the correction amount is applied to the color of a chewing check gum image to detect the true color of the chewing check gum placed on the dedicated mount 10. The true color of the post-chew chewing check gum is applied to a preliminarily stored chewing ability determination color scale to measure the chewing ability of a person who has chewed the chewing check gum.

## Description

### TECHNICAL FIELD

The present invention relates to a method of measuring a chewing ability by using a chewing check gum, a chewing ability confirmation system, and a dedicated mount on which the chewing check gum is placed.

### BACKGROUND ART

In recent years, with an increasing concern about healthy lifespan, attention is focused on "chewing ability" which is one of barometers of the healthy lifespan. This creates the need for a method of readily determining the chewing ability. Although the determination of the chewing ability can be achieved by using a dedicated device, more convenient means is "chewing check gum" ("xylitol chewing check gum" (NPL 1)). The chewing check gum is designed so that the color thereof is changed from green to red as the chewing proceeds in oral cavity. Therefore, the degree of the color change can be used for the determination of the chewing ability.

Exemplary methods of reflecting the degree of the color change in the chewing ability include: a method such that the color of a post-chew gum is visually determined with reference to a five-level or ten-level color scale (see PTL 1 and PTL 2); and a method such that the color of a post-chew gum is objectively measured with the use of an instrument called "color difference meter."

In the former method, the color of the post-chew gum is visually compared with color samples and, therefore, a problem is lack of objectivity and accuracy. In the latter method, the color difference meter to be used for the measurement is very expensive and, therefore, a problem is that the color difference meter cannot be readily used.

### CITATION LIST

### PATENT LITERATURE

PTL 1: JP5443927 (which describes a method of preparing a color scale for determination of a chewing ability)
PTL 2: JP5843567 (which describes a method of preparing a color scale for determination of a chewing ability)

### NON-PATENT LITERATURE

NPL 1: oralcare.co.jp/product/post-32.html

### SUMMARY OF INVENTION

### PROBLEMS TO BE SOLVED BY INVENTION

It is an object of the present invention to provide a method and an apparatus that make it possible to objectively, accurately and readily measure the color of a post-chew gum. It is another object of the present invention to provide a method and an apparatus of determining a chewing ability based on the measured gum color.

### SOLUTION TO PROBLEMS

A post-chew gum, which is placed on a dedicated mount, is photographed and analyzed with the use of a camera and an image analysis application such as of a smartphone, and a chewing ability is determined based on the color of the gum.

Specifically, the post-chew gum is first placed on a mount on which a color chart including print primary colors and a gum placement position are shown. Subsequently, the mount and the gum on the mount are photographed by the camera such as of the smartphone. Then, the chewing ability is determined by analyzing an image of the photographed gum with the use of the application and calculating the degree of the color change of the gum.

Features of the mount include chromatic reference colors (color chart), a lightness correction gray color and a gum position detection marker provided on the mount.

The color of the photographed gum can be analyzed with the use of the chromatic reference colors (color chart). Further, the gray color is provided around the chromatic reference colors (color chart), making it possible to correct the illuminance unevenness of the chromatic reference colors (color chart) (lighting conditions and the like). Thus, the lightness of the chromatic reference colors (color chart) can be accurately corrected. Further, the provision of the gum position detection marker makes it possible to accurately detect the position of the mounted (placed) gum and to accurately analyze the color of the gum.

An analysis mechanism can be embodied by a process based on a dedicated program or application software.

### EFFECT OF INVENTION

According to the present invention, the color of the post-chew gum can be objectively, accurately and readily measured by photographing the gum placed on the measurement mount by the camera such as of the smartphone and analyzing the image of the gum. Then, the chewing ability can be determined based on the color of the post-chew gum.

With reference to an image correction process performed on the image of the chromatic reference colors (color chart) in image data of the chromatic reference colors (color chart) and the post-chew gum contained in a single image, specifically, the accurate color of the gum can be detected in consideration of an image correction process performed on the image of the gum. Thus, the chewing ability can be determined.

### BRIEF DESCRIPTION OF DRAWINGS

FIG. 1 is a diagram showing a dedicated mount according to one example embodiment of the present invention.
FIG. 2 is a configuration block diagram of a chewing ability confirmation system according to the example embodiment of the present invention.
FIG. 3 is a flowchart showing a process to be performed by the chewing ability confirmation system according to the example embodiment of the present invention.
FIG. 4 is a screen image diagram of a chewing ability determination result to be received by a user.

### DESCRIPTION OF EXAMPLE EMBODIMENT

With reference to the drawings, one example embodiment of the present invention will hereinafter be described specifically.

In the following detailed description of the example embodiment of the present invention, "xylitol chewing check gum (trade name)" is used as a chewing check gum by way of example. Xylitol chewing check gum (trade name) is a gum specially designed to be originally green in an unchewed state and to be gradually changed from green to red as the pH thereof increases from an acid pH to an alkali pH by sufficient chewing thereof.

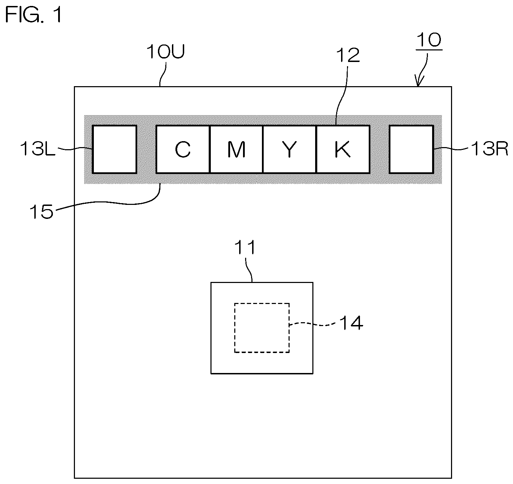

FIG. 1 is a plan view showing an exemplary dedicated mount on which post-chew xylitol chewing check gum (trade name) obtained, for example, after being chewed 60 times is placed.

The dedicated mount 10 includes, for example, a sheet made of paper (cardboard), a plastic plate, a metal plate or the like. The dedicated mount 10 has a generally square shape by way of example, and a gum placement position 11 in which a post-chew chewing check gum (hereinafter referred to simply as "gum") is placed is defined in a center portion of the dedicated mount 10. The dedicated mount 10 includes chromatic reference colors (color chart) 12 printed, for example, on a region thereof extending transversely alongside an upper edge 10U thereof. The color chart 12 includes reference colors to be used for detection of the color of the gum placed in the gum placement position 11. Specifically, the color chart 12 includes four color blocks, i.e., a cyan block C, a magenta block M, a yellow block Y and a black block K, arranged transversely (laterally) alongside the upper edge 10U of the dedicated mount 10. In the drawings attached herein, the four color blocks are not colored because color notation is not common, but are indicated by characters C, M, Y, K.

The dedicated mount 10 further includes gum position detection markers 13L, 13R provided on the left side and the right side of the color chart 12. The gum position detection markers 13L, 13R are required for identification of a gum detection position, so that a gum placement position on the dedicated mount 10, i.e., the gum placed on the dedicated mount 10, can be accurately detected. The gum position detection markers 13L, 13R make it possible to identify, for example, a region 14 indicated by a broken line as a detection region to detect a color in the detection region 14 (i.e., the color of the gum).

In this example embodiment, the two gum position detection markers 13L, 13R are disposed in spaced relation, whereby the position detection accuracy of the detection region 14 can be improved. In addition, the positions of the color chart 12 and a gray color 15 can be accurately detected.

On the dedicated mount 10 according to this example embodiment, the two gum position detection markers 13L, 13R are provided on the left side and the right side of the color chart 12 by way of example, but three or four gum position detection markers may be provided on the mount 10. Alternatively, a single gum position detection marker may be provided depending on the indication of the marker.

Examples of the gum position detection markers include two-dimensional codes such as QR codes (registered trademark) .

On the dedicated mount 10, the gray color 15 is provided in a peripheral region surrounding the color chart 12 and the gum position detection markers 13L, 13R. The gray color 15 is provided for correction of the illuminance unevenness (lighting conditions and the like) of the color chart 12 when the color chart 12 is read. The provision of the gray color 15 makes it possible to read the color chart 12 and the color of the gum based on uniform lightness. This improves the detection accuracy of the gum color.

In this example embodiment, the gray color 15 is provided around the gum position detection markers 13L, 13R as well, but may be provided at least around the color chart 12.

In this example embodiment, an achromatic gray color is provided as the gray color 15 by way of example. Instead of the achromatic gray color, a single-illuminance determination reference color, e.g., a (chromatic) single color having a moderate luminous intensity or an intermediate luminous intensity, may be used.

On a modification of the dedicated mount 10, a specific QR code (registered trademark) may be provided, for example, in the gum placement position 11. The dedicated mount 10 may be designed so that a web application can be actuated to perform a process between a smartphone 30 and a server 40 by reading the QR code (registered trademark) by means of a camera 31 of the smartphone 30 to be described later.

Further, a preferred form of the dedicated mount 10 is such that the color chart 12, the gum position detection markers 13L, 13R and the gray color 15 are disposed on one side of a mount surface, e.g., in a region closer to one edge (specifically, the upper edge 10U) of the mount surface, and the gum placement position is defined in the rest of the mount surface, whereby the gum placement position is prevented from interfering with the color chart 12, the gum position detection markers 13L, 13R and the gray color 15 for easy positioning of the gum and for easy use.

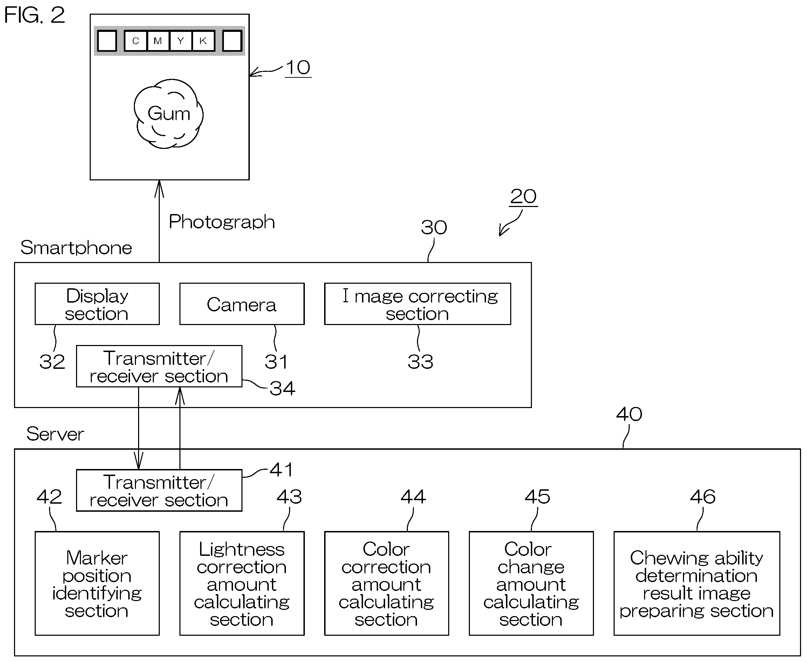

FIG. 2 is a configuration block diagram of a chewing ability confirmation system according to the example embodiment of the present invention.

The chewing ability confirmation system 20 can be constituted by a smartphone 30 and a server 40 by way of example.

The smartphone 30 generally includes a camera 31, a display section 32 capable of displaying an image photographed by the camera 31 and the like, and an image correcting section 33 capable of correcting the image photographed by the camera 31. The image correcting section 33 is sometimes based on hardware, but is typically based on software capable of performing a model-specific image correction process uniquely designed for the smartphone 30.

Further, the smartphone 30 includes a transmitter/receiver section 34 that transmits and receives data such as image data (for communications).

The smartphone 30 is installed with a specific smartphone application or actuates a specific web application, as described above, to thereby communicate with the server 40 to cause the server 40 to perform a predetermined process and acquire the result of the process.

In this example embodiment, the smartphone 30 is used by way of example but not by way of limitation. Alternatively, any of various information processing terminals such as a PC (personal computer) having a camera function or a tablet may be used for the communication with the server 40.

The server 40 includes various functional processing sections such as a transmitter/receiver section 41 for the communication with the smartphone 30 on a client side (user), a marker position identifying section 42, a lightness correction amount calculating section 43, a color correction amount calculating section 44, a color change amount calculating section 45 and a chewing ability determination result image preparing section 46. The functions of these functional processing sections 41 to 46 may be embodied based on data and a predetermined program (software) preliminarily stored in the server 40.

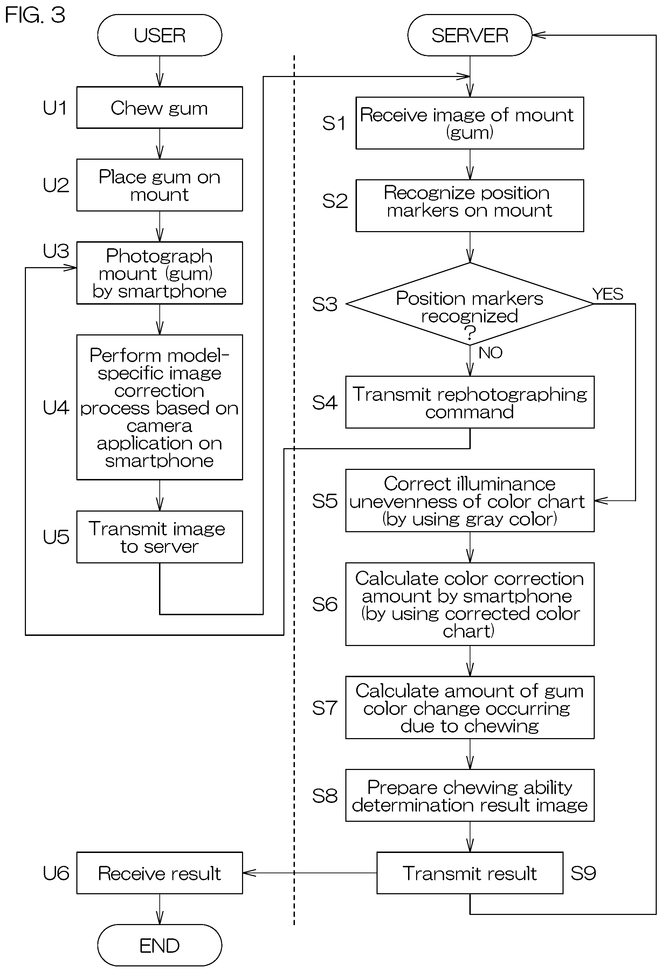

FIG. 3 is a flowchart showing a process to be performed by the chewing ability confirmation system 20 shown in FIG. 2 according to the example embodiment of the present invention. In FIG. 3, the left half of the flowchart shows a process sequence to be performed on the user side (including process steps to be humanly performed by the user), and the right half of the flowchart shows a process sequence to be performed in the server (i.e., a process sequence to be performed based on the program).

Referring to FIG. 3, the user first chews xylitol chewing check gum (trade name), for example, 60 times (Step U1). Then, the post-chew gum is placed in the gum placement position 11 on the dedicated mount 10 (Step U2).

Subsequently, the smartphone application or the web application for the chewing ability check is actuated, and then the dedicated mount 10 on which the gum is placed is photographed by the camera of the smartphone 30 (Step U3).

Where the specific QR code (registered trademark) is provided in the gum placement position 11 of the dedicated mount 10 as in the aforementioned example, the specific QR code (registered trademark) may be read to actuate the web application before the gum is placed.

When the dedicated mount 10 on which the gum is placed is photographed by the camera of the smartphone 30, the gum is photographed together with the color chart 12, the gum position detection markers 13L, 13R and the gray color 15 provided on the dedicated mount 10.

Upon the photographing by the camera 31 of the smartphone 30, the smartphone 30 sometimes automatically preforms the model-specific image correction process on the photographed image based on a camera application (Step U4).

Even if the image correction process is performed, however, the entire photographed image is simply subjected to the image correction process, but different image correction processes are not performed separately on the image of the color chart 12 and on the image of the gum. Therefore, the image correction process never influences the detection of the color of the gum according to the example embodiment of the present invention.

The smartphone 30 transmits the image data of the dedicated mount 10 photographed by the camera 31 to the server 40 (Step U5).

Upon receiving the image data of the dedicated mount 10 through the smartphone application or the web application (Step S1), the server 40 recognizes the image of the gum position detection markers 13L, 13R in the image data (Step S2). If the image of the gum position detection markers 13L, 13R cannot be recognized in the image data (NO in Step S3), the server 40 transmits a rephotographing command to the smartphone 30 to rephotograph the dedicated mount 10 (Step S4).

If the image of the gum position detection markers 13L, 13R can be recognized in the image data (YES in Step S3), on the other hand, it is determined that the entire necessary image of the dedicated mount 10 is photographed. With reference to FIG. 1, more specifically, if the gum position detection markers 13L, 13R can be recognized in the image of the photographed dedicated mount 10, it is determined that the images of the color chart 12 and the gray color 15 present between the gum position detection markers 13L, 13R and the image of the detection region 14 present in a middle portion below the gum position detection markers 13L, 13R are contained in the image of the photographed mount.

Then, the server 40 identifies an image region 12 in which the color chart 12 is shown, based on the positions of the gum position detection markers 13L, 13R, and detects the RGB values of CMYK color markers in the image region 12. Further, the server 40 identifies an image region 15 in which the gray color 15 is shown, based on the positions of the gum position detection markers 13L, 13R, and detects the luminous intensity value of the gray color in the image region 15 (e.g., a luminous intensity value indicating one of 256 levels defined on an 8-bit basis).

Then, the detected RGB values of the CMYK color markers are corrected by using the luminous intensity value of the gray color provided in the vicinity of the CMYK color markers, whereby the illuminance unevenness of the color chart 12 is corrected (Step S5).

That is, in Step S5, the RGB values of the CMYK color markers in the image region 12 in which the color chart 12 is shown are corrected with the use of the luminous intensity value of the gray color in order to eliminate the influence of a light source from the RGB values of the CMYK color markers (in order to cancel the influence of the light source) .

Subsequently, the server 40 calculates the amount of color correction performed by the smartphone 30 (Step S6). The RGB values of the CMYK color markers corrected in Step S5 are used for the calculation of the color correction amount.

To what degree and how the color correction is performed by the smartphone 30 can be calculated by comparing the image of the photographed color chart 12 with the color chart 12 printed on the dedicated mount 10. Then, differences (correction amounts) in hue, lightness, chroma and the like between the color chart 12 printed on the dedicated mount 10 and the image of the color chart 12 photographed by the smartphone 30 can be calculated, for example, as a correction formula.

Further, the server 40 detects an image observed in the detection region 14 of the dedicated mount 10 (i.e., the image of the gum) based on the positions of the gum position detection markers 13L, 13R. The image of the photographed color chart 12 and the image of the photographed gum are slightly different from each other in lightness and the like, because the color chart 12 and the gum are different from each other in physical properties. The smartphone 30 performs the color correction on the image of the gum in the same manner as on the image of the color chart 12. Therefore, the server 40 applies the calculated correction formula to the color of the detected gum image to detect the correct color (true color) of the gum (Step S6).

Then, the true color of the gum thus detected is compared with a preliminarily registered reference color (the color of unchewed xylitol chewing check gum), whereby the amount of the color change of the gum occurring due to the chewing is calculated (Step S7).

Subsequently, a chewing ability determination result image is prepared by applying the calculated color change amount to a color scale preliminarily registered for the determination of xylitol gum chewing ability (Step S8).

The chewing ability determination result image thus prepared is transmitted to the smartphone 30 (Step S9).

The user receives the chewing ability determination result image on the smartphone 30 (Step U6). Thus, the user can find his or her chewing ability, which is advantageously used to control his or her future lifestyle habits and the like.

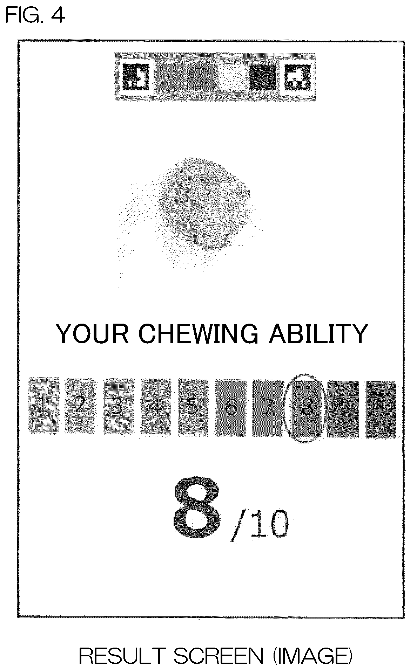

FIG. 4 shows a screen image of the chewing ability determination result to be received by the user for reference.

In the example embodiment described above with reference to FIGS. 2 and 3, the chewing ability confirmation system is constituted by the smartphone 30 and the server 40.

Alternatively, the chewing ability confirmation system according to the present invention may include the smartphone 30 alone. In this case, the functional processing sections such as the marker position identifying section 42, the lightness correction amount calculating section 43, the color correction amount calculating section 44, the color change amount calculating section 45 and the chewing ability determination result image preparing section 46 provided in the server 40 shown in FIG. 2 are embodied on a software basis by preliminarily storing data in the smartphone 30 and installing a predetermined program (software) in the smartphone 30.

Then, a software process to be performed by the smartphone 30 is a combination of process sequences excluding Steps U5 and U6 and Steps S1 and S9 from the process sequences to be respectively performed by the user and the server as shown in FIG. 3.

In the example embodiment described above, the amount of the color change of the gum occurring due to the chewing is calculated in Step S7, but the calculation of the color change amount may be omitted. For the omission of the calculation of the color change amount, the level of the chewing ability may be determined or judged based on the post-chew gum color, for example, by applying the true color of the post-chew gum to a parameter of a relationship between the chewing ability and the gum color change, because the true gum color changed due to the chewing is detected in Step S6.

The present invention is not limited to the example embodiment described above. The features of the present invention based on the scope of the present invention are as follows:

The present invention provides a chewing ability measurement method of measuring a chewing ability by using a chewing check gum, the method including the steps of: placing a post-chew chewing check gum on a dedicated mount 10 provided with chromatic reference colors 12; photographing the dedicated mount 10 on which the chewing check gum is placed by a camera, and forming an image containing an image of the chromatic reference colors 12 and an image of the chewing check gum together; comparing the image of the chromatic reference colors 12 contained in the formed image with the chromatic reference colors 12 provided on the dedicated mount 10 to calculate a color correction amount of the chromatic reference color image contained in the formed image; applying the calculated color correction amount to a color of the chewing check gum image contained in the formed image to detect a true color of the chewing check gum placed on the dedicated mount 10; and comparing the detected true color of the chewing check gum with a color of an unchewed chewing check gum preliminarily registered, and calculating a color change amount of the gum occurring due to chewing.

The present invention also provides a chewing ability measurement method of measuring a chewing ability by using a chewing check gum, the method including the steps of: placing a post-chew chewing check gum on a dedicated mount 10 provided with chromatic reference colors 12; photographing the dedicated mount 10 on which the chewing check gum is placed by a camera, and forming an image containing an image of the chromatic reference colors 12 and an image of the chewing check gum together; comparing the image of the chromatic reference colors 12 contained in the formed image with the chromatic reference colors 12 provided on the dedicated mount 10 to calculate a color correction amount of the chromatic reference color image contained in the formed image; applying the calculated color correction amount to a color of the chewing check gum image contained in the formed image to detect a true color of the chewing check gum placed on the dedicated mount 10; and applying the detected true color of the chewing check gum to a preliminarily stored chewing ability determination color scale to thereby determine a chewing ability of a person who has chewed the chewing check gum, and outputting the chewing ability of the person.

In the chewing ability measurement method according to the present invention, the dedicated mount 10 includes a single-illuminance determination reference color provided in the vicinity of the chromatic reference colors, and the step of calculating the color correction amount of the chromatic reference color image includes the step of correcting illuminance unevenness of the chromatic reference colors 12 based on lightness of the single-illuminance determination reference color.

In the present invention, an achromatic gray color may be used as the single-illuminance determination reference color.

In the chewing ability measurement method according to the present invention, the dedicated mount 10 is provided with position detection markers 13L, 13R, and the method includes the step of detecting the image of the chromatic reference colors 12 and the image of the chewing check gum in the image formed by photographing the dedicated mount 10 by the camera, based on an image of the position detection markers 13L, 13R.

Further, the present invention provides a chewing ability confirmation system of checking a chewing ability by using a chewing check gum, the system including: a camera that photographs a dedicated mount 10 provided with chromatic reference colors 12 with a post-chew chewing check gum placed in a predetermined position of the dedicated mount 10; specific image extracting means that extracts an image of the chromatic reference colors 12 and an image of the chewing check gum from an image formed by photographing the dedicated mount 10 by the camera; correction amount calculating means that compares the chromatic reference color image extracted by the specific image extracting means with the chromatic reference colors 12 of the dedicated mount 10 to calculate an amount of color correction performed on the chromatic reference color image; color detecting means that corrects a color of the chewing check gum image extracted by the specific image extracting means by applying the color correction amount calculated by the correction amount calculating means to the color of the chewing check gum image to detect a true color of the chewing check gum placed on the dedicated mount 10; and outputting means that applies the true color of the chewing check gum detected by the color detecting means to a preliminarily stored chewing ability determination color scale to thereby determine a chewing ability of a person who has chewed the chewing check gum, and outputs the chewing ability of the person.

In the chewing ability confirmation system according to the present invention, the dedicated mount 10 is provided with position detection markers 13L, 13R for identification of positions of images to be extracted, and the specific image extracting means identifies positions of the chromatic reference color image and the chewing check gum image to be extracted, based on an image of the position detection markers in the image formed by photographing the dedicated mount 10 by the camera, and extracts the images in the identified positions.

The chewing ability confirmation system according to the present invention includes an information processing terminal having a camera function, and the camera is embodied by the camera function of the information processing terminal. The specific image extracting means, the correction amount calculating means, the color detecting means and the outputting means are embodied by a program installed in the information processing terminal.

The chewing ability confirmation system according to the present invention includes an information processing terminal having a camera function and a server, and the camera is embodied by the camera function of the information processing terminal. The specific image extracting means, the correction amount calculating means, the color detecting means and the outputting means are embodied by a program installed in the server.

The present invention also provides a dedicated mount 10 to be used to measure a chewing ability by using a chewing check gum. The dedicated mount 10 has a region predefined on a surface of the mount as a position in which a post-chew chewing check gum is placed, and includes chromatic reference colors 12 printed in a position different from the predefined region on the surface of the mount. The dedicated mount 10 further includes position detection markers 13L, 13R provided for identification of the predefined region in association with the position in which the chromatic reference colors 12 are printed on the mount.

The dedicated mount according to the present invention includes a gray color printed around the chromatic reference colors printed on the mount.

The dedicated mount according to the present invention may include a sheet of paper, plastic or metal.

In the dedicated mount according to the present invention, the chromatic reference colors, the position detection markers and the gray color are provided in a surface region closer to one edge of the dedicated mount, and the position in which the chewing check gum is placed is set in a surface region of the mount other than the region closer to the one edge.

### DESCRIPTION OF REFERENCE CHARACTERS

- 10:: Dedicated mount
- 11:: Gum placement position
- 12:: Chromatic reference colors
- 13L, 13R:: Position detection markers
- 14:: Detection region
- 15:: Gray color
- 20:: Chewing ability confirmation system
- 30:: Smartphone
- 31:: Camera
- 32:: Display section
- 33:: Image correcting section
- 34:: Transmitter/receiver section
- 40:: Server
- 41:: Transmitter/receiver section
- 42:: Marker position identifying section
- 43:: Lightness correction amount calculating section
- 44:: Color correction amount calculating section
- 45:: Color change amount calculating section
- 46:: Chewing ability determination result image preparing section

## Claims

1. A chewing ability measurement method of measuring a chewing ability by using a chewing check gum, the method comprising the steps of:
placing a post-chew chewing check gum on a dedicated mount provided with a chromatic reference color;
photographing the dedicated mount on which the chewing check gum is placed by a camera, and forming an image containing an image of the chromatic reference color and an image of the chewing check gum together;
comparing the chromatic reference color image contained in the formed image with the chromatic reference color provided on the dedicated mount to calculate a color correction amount of the chromatic reference color image contained in the formed image;
applying the calculated color correction amount to a color of the chewing check gum image contained in the formed image to detect a true color of the chewing check gum placed on the dedicated mount; and
comparing the detected true color of the chewing check gum with a color of an unchewed chewing check gum preliminarily registered, and calculating a color change amount of the gum occurring due to chewing.

2. A chewing ability measurement method of measuring a chewing ability by using a chewing check gum, the method comprising the steps of:
placing a post-chew chewing check gum on a dedicated mount provided with a chromatic reference color;
photographing the dedicated mount on which the chewing check gum is placed by a camera, and forming an image containing an image of the chromatic reference color and an image of the chewing check gum together;
comparing the chromatic reference color image contained in the formed image with the chromatic reference color provided on the dedicated mount to calculate a color correction amount of the chromatic reference color image contained in the formed image;
applying the calculated color correction amount to a color of the chewing check gum image contained in the formed image to detect a true color of the chewing check gum placed on the dedicated mount; and
applying the detected true color of the chewing check gum to a preliminarily stored chewing ability determination color scale to thereby determine a chewing ability of a person who has chewed the chewing check gum, and outputting the chewing ability of the person.

3. The chewing ability measurement method according to claim 1 or 2,
wherein the dedicated mount includes a single-illuminance determination reference color provided in vicinity of the chromatic reference color thereon,
wherein the step of calculating the color correction amount of the chromatic reference color image includes the step of correcting illuminance unevenness of the chromatic reference color based on lightness of the single-illuminance determination reference color.

4. The chewing ability measurement method according to claim 3, wherein the single-illuminance determination reference color provided on the dedicated mount comprises a gray color.

5. The chewing ability measurement method according to claim 1 or 2,
wherein the dedicated mount includes a position detection marker provided thereon,
the method comprising the step of detecting the chromatic reference color image and the chewing check gum image in the image formed by photographing the dedicated mount by the camera, based on an image of the position detection marker.

6. A chewing ability confirmation system of checking a chewing ability by using a chewing check gum, the system comprising:
a camera that photographs a dedicated mount provided with a chromatic reference color with a post-chew chewing check gum placed in a predetermined position of the dedicated mount;
specific image extracting means that extracts an image of the chromatic reference color and an image of the chewing check gum from an image formed by photographing the dedicated mount by the camera;
correction amount calculating means that compares the chromatic reference color image extracted by the specific image extracting means with the chromatic reference color provided on the dedicated mount to calculate an amount of color correction performed on the chromatic reference color image;
color detecting means that corrects a color of the chewing check gum image extracted by the specific image extracting means by applying the color correction amount calculated by the correction amount calculating means to the color of the chewing check gum image to detect a true color of the chewing check gum placed on the dedicated mount; and
outputting means that applies the true color of the chewing check gum detected by the color detecting means to a preliminarily stored chewing ability determination color scale to thereby determine a chewing ability of a person who has chewed the chewing check gum, and outputs the chewing ability of the person.

7. The chewing ability confirmation system according to claim 6,
wherein the dedicated mount includes a position detection marker provided thereon for identification of positions of images to be extracted,
wherein the specific image extracting means identifies positions of the chromatic reference color image and the chewing check gum image to be extracted, based on an image of the position detection marker in the image formed by photographing the dedicated mount by the camera, and extracts the images in the identified positions.

8. The chewing ability confirmation system according to claim 6 or 7, comprising an information processing terminal having a camera function,
wherein the camera is embodied by the camera function of the information processing terminal,
wherein the specific image extracting means, the correction amount calculating means, the color detecting means and the outputting means are embodied by a program installed in the information processing terminal.

9. The chewing ability confirmation system according to claim 6 or 7, comprising an information processing terminal having a camera function, and a server,
wherein the camera is embodied by the camera function of the information processing terminal,
wherein the specific image extracting means, the correction amount calculating means, the color detecting means and the outputting means are embodied by a program installed in the server.

10. A dedicated mount to be used to measure a chewing ability by using a chewing check gum, the dedicated mount comprising:
a region predefined on a surface of the mount as a position in which a post-chew chewing check gum is placed;
a chromatic reference color printed in a position different from the predefined region on the surface of the mount; and
a position detection marker provided for identification of the predefined region in association with the position in which the chromatic reference color is printed on the mount.

11. The dedicated mount according to claim 10, further comprising a gray color printed around the chromatic reference color printed on the mount.

12. The dedicated mount according to claim 10 or 11, comprising a sheet of paper, plastic or metal.

13. The dedicated mount according to claim 11, wherein the chromatic reference color, the position detection marker and the gray color are provided in a surface region closer to one edge of the mount, and the position in which the chewing check gum is placed is set in a surface region of the mount other than the region closer to the one edge.
